(19) Europäisches Patentamt
European Patent Office
Office européen des brevets

(11) Veröffentlichungsnummer: **0 066 196**
B1

(12) # EUROPÄISCHE PATENTSCHRIFT

(45) Veröffentlichungstag der Patentschrift: **08.08.84**

(51) Int. Cl.³: **C 07 D 211/14,** C 07 D 265/30, C 07 D 295/02, A 01 N 43/00

(21) Anmeldenummer: **82104392.4**

(22) Anmeldetag: **19.05.82**

(54) **Cyclohexenderivate, Verfahren zu ihrer Herstellung und diese enthaltende Fungizide.**

(30) Priorität: **29.05.81 DE 3121349**

(43) Veröffentlichungstag der Anmeldung: **08.12.82 Patentblatt 82/49**

(45) Bekanntmachung des Hinweises auf die Patenterteilung: **08.08.84 Patentblatt 84/32**

(84) Benannte Vertragsstaaten: **AT BE CH DE FR GB IT LI NL SE**

(56) Entgegenhaltungen:
**EP - A - 0 008 686**
**EP - A - 0 019 769**
**EP - A - 0 032 673**
**DE - A - 2 656 747**
**DE - A - 2 752 096**
**DE - A - 2 752 135**
**US - A - 3 423 406**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Gramlich, Walter, Dr., Auf der Hoehe11, D-6803 Edingen (DE)**
Erfinder: **Himmele, Walter, Dr., Eichenweg 14, D-6909 Walldorf (DE)**
Erfinder: **Martin, Christoph, Dr., Kolpingstrasse 6, D-6800 Mannheim (DE)**
Erfinder: **Pommer, Ernst-Heinrich, Dr., Berliner Platz 7, D-6703 Limburgerhof (DE)**
Erfinder: **Siegel, Hardo, Dr., Hans-Purrmann-Allee 25, D-6720 Speyer (DE)**

**EP 0 066 196 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue wertvolle 3-Cyclohexen-3'-yl-2-methyl-propyl-1-cycloalkylaminderivate, Verfahren zu ihrer Herstellung, diese enthaltende Fungizide und Verfahren zur Bekämpfung von Pilzen.

Es ist bekannt, daß man 4-[3-(p-tert.Butyl-phenyl)-2-methyl-propyl]-cis-2,6-Dimethyl-morpholin (DE-OS 2 656 747, DE-OS 2 752 135) für die Bekämpfung von Mehltau und Rostpilzen im Getreide speziell im Weizen, verwenden kann. Anstelle des 2,6-Dimethylmorpholin-Restes lassen sich auch Verbindungen mit Resten anderer cyclischer Amine wie Piperidin oder 3-Methyl-piperidin verwenden, oder Verbindungen, die anstelle des tert.Butylphenyl-Restes den tert.Butyl-cyclohexylrest enthalten. Die Dauer der Wirksamkeit dieser Verbindungen, innerhalb der ein voller Schutz gegen die Schadpilze erzielt wird, liegt bei drei bis vier Wochen. Eine Wiederholung der Anwendung des Pilzbekämpfungsmittels ist oft erforderlich.

In der DE-OS 3 001 581 werden fungizid wirksame N-[3-(4'-tert.Butylcyclohex-1'en-1'yl)-2-methyl-propyl-1]-cyclo-alkylamine beschrieben, die man durch partielle Hydrierung der in den DE-OS 2 656 747 und DE-OS 2 752 135 beschriebenen entsprechenden aromatischen Verbindungen erhält. Die Ausbeute liegt bei dieser Herstellungsweise jedoch nur bei ca. 20%, die Abtrennung der partiell hydrierten Produkte von den aromatischen Ausgangsverbindungen sowie von den gleichzeitig entstehenden perhydrierten cis- und trans-Formen erfordert einen sehr hohen Aufwand (Destillation). Bei den so erhaltenen Cyclohexenverbindungen der Formel IV

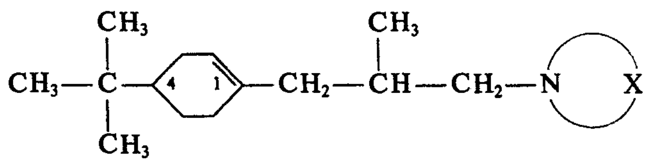

(IV)

liegt die Doppelbindung am C-Atom 1.

Es wurde nun gefunden, daß Cyclohexenderivate der Formel I

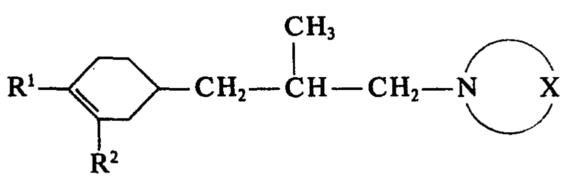

(I)

in der $R^1$ und $R^2$ voneinander verschieden sind und Wasserstoff oder eine tert. Butylgruppe bedeuten und N und X Glieder eines sechsgliedrigen heterocyclischen Ringes bedeuten, wobei X eine Alkylenkette mit fünf Gliedern ist, die gegebenenfalls ein Sauerstoffatom als Kettenglied enthält, und deren Alkylenreste gegebenenfalls ein- oder mehrfach durch Alkylreste mit 1 bis 4 Kohlenstoffatomen oder durch den Rest $-CH_2OH$ oder sein Acetyl- oder Propionylderivat substituiert sind, und ihre Salze eine gute fungizide Wirkung haben.

Ein Alkylenrest ist beispielsweise der Methylenrest. Alkylreste mit 1 bis 4 C-Atomen sind beispielsweise Methyl, Ethyl, Propyl, iso-Butyl, sec.Butyl, n-Butyl. Salze sind beispielsweise nicht-phytotoxische Salze mit anorganischen oder organischen Säuren, z. B. Halogenwasserstoffsäuren, beispielsweise Salzsäure, Bromwasserstoffsäure, oder Mineralsäuren, beispielsweise Schwefelsäure, Phosphorsäure, oder Carbonsäuren, beispielsweise Essigsäure, Propionsäure.

Die Bestimmung, daß $R^1$ und $R^2$ voneinander verschieden sind, bedeutet, daß in einer konkreten Verbindung $R^1$ und $R^2$ nicht gleichzeitig Wasserstoff oder nicht gleichzeitig je eine tert.Butylgruppe bedeuten.

Die neuen Verbindungen werden in einfacher Weise und in sehr guten Ausbeuten erhalten, wenn man Verbindungen der Formel II

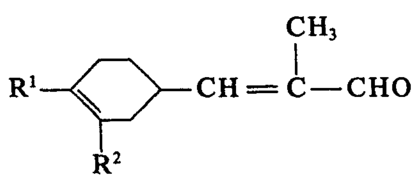

(II)

in der $R^1$ und $R^2$ die oben genannten Bedeutungen haben, in Gegenwart eines Amins der Formel III

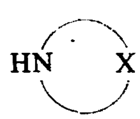

(III)

in der N und X die oben genannten Bedeutungen haben, in Gegenwart eines Hydrierkatalysators mit Wasserstoff umsetzt und die so erhaltene Verbindung gegebenenfalls in ihr Salz überführt.

Die Verbindungen der Formel II sind beispielsweise auf folgendem Wege zugänglich

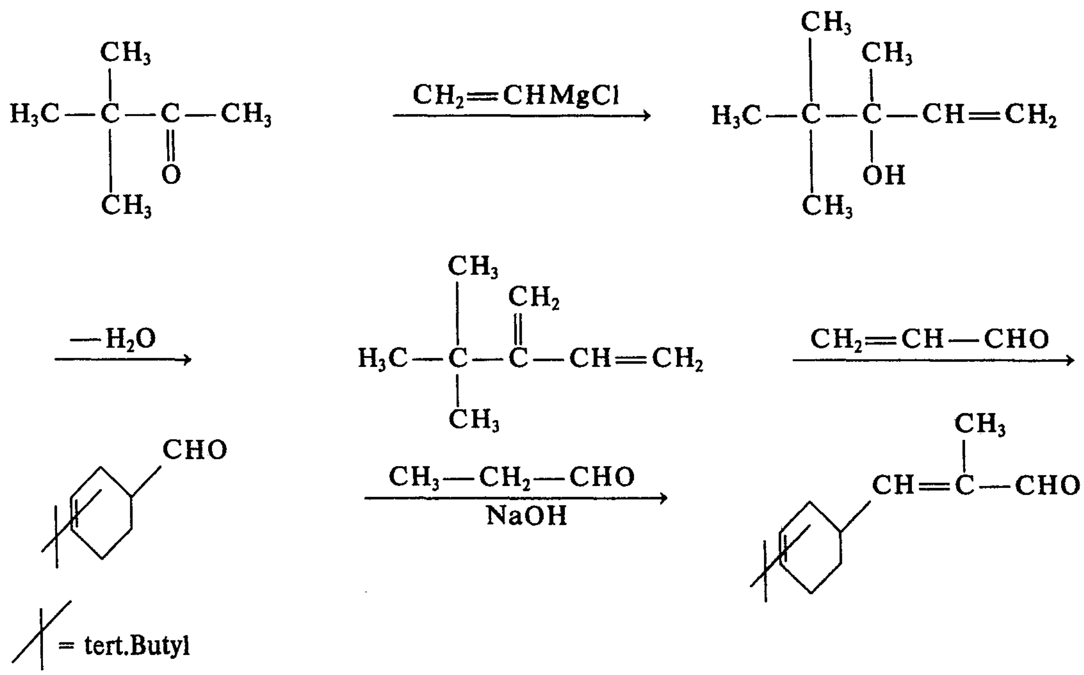


Überraschend verbleibt bei der weiteren Umsetzung mit Wasserstoff und mit Aminen der Formel III in Gegenwart eines Hydrierkatalysators zu den Verbindungen der Formel I die Doppelbindung im Cyclohexenring an der bezeichneten Stelle vollständig erhalten, während die Doppelbindung in der Seitenkette hydriert wird. Geeignete Amine sind z. B. Pyrrolidin, Morpholin, Thiomorpholin, 2, 6-Dimethylmorpholin (cis/trans-Gemisch), cis-2,6-Dimethylmorpholin, 2,5-Dimethylmorpholin, Piperidin, 3,5-Dimethylpiperidin, 3-Methylpiperidin, 3,4-Dimethylpiperidin, 3-Ethyl-4-methylpiperidin, 3-Methyl-4-ethylpiperidin, 2,6-Dimethylpiperidin, Hexamethylenimin, 2-Methyl-hexamethylenimin, 3-Methyl-hexamethylenimin. Als Katalysatoren für die Umsetzung eignen sich besonders Palladium und Rhodium, bevorzugt jedoch Palladium auf einem inerten Träger wie $Al_2O_3$, $TiO_2$, Kohle usw. Auch natürliche oder künstliche Silikate wie Aluminium-Silikate oder Magnesium-Silikat sind als Träger geeignet.

Die Reaktionstemperatur liegt bei 25 bis 180° C, bevorzugt 30 bis 110° C. Der Wasserstoffdruck liegt bei 2 bis 100 bar.

Die Verbindungen der Formel I enthalten ein asymmetrisches Kohlenstoffatom. Die Erfindung umfaßt sowohl die Racemate als auch die einzelnen optisch aktiven Formen.

Man erhält sie, indem man die fungizid wirksamen Amine der Formel I in an sich bekannter Weise mit optisch aktiven Säuren voneinander trennt. Ein weiteres vorteilhaftes Verfahren besteht darin, daß man die Verbindungen der Formel II

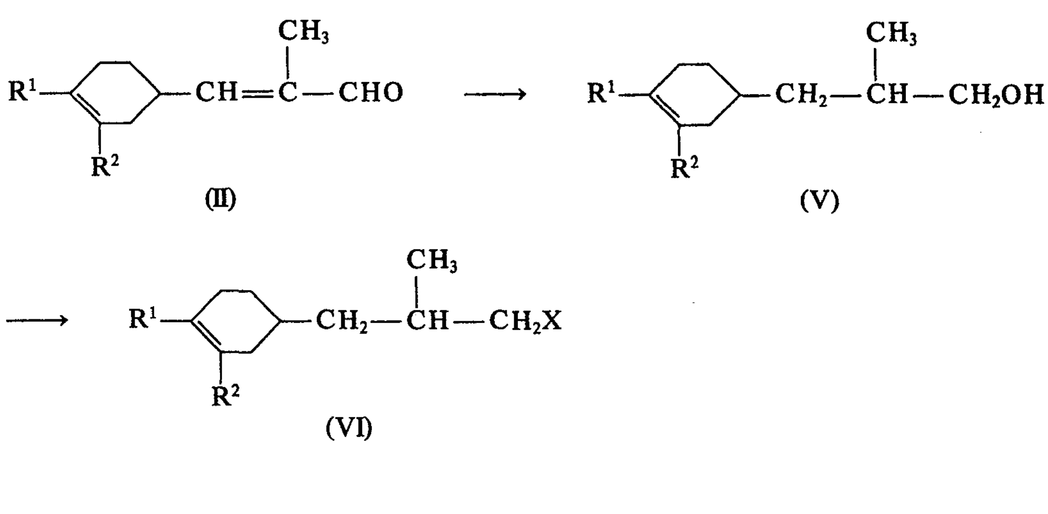

(II)

(V)

(VI)

mit Hefe in einem Fermenter zu dem optisch aktiven Alkohol V reduziert. Hieraus läßt sich nach bekannten Methoden die Verbindung VI herstellen, wobei X ein Halogen wie Chlor oder Brom oder einen Tosyl- oder Mesylrest bedeutet. In den Formeln II, V und VI haben $R^1$ und $R^2$ die oben genannten Bedeutungen. Die Verbindungen der Formel VI lassen sich in an sich bekannter Weise mit Aminen der Formel III zu den fungizid wirksamen Verbindungen der Formel I umsetzen.

Die folgenden Vorschriften und Beispiele erläutern die Herstellung der Vorprodukte und der Endprodukte.

## Vorschrift A

### tert.Butylbutadien

Zu 1 Mol Methyl-vinyl-tert. butylcarbinol, in bekannter Weise hergestellt aus einer Vinylmagnesiumchloridlösung in Tetrahydrofuran und Methyl-tert.butylketon, gibt man 30 g Oxalsäure, 50 ml Wasser und 2 g Hydrochinon. Man erhitzt 30 min unter Rückfluß und destilliert dann das entstandene tert.Butylbutadien zusammen mit Wasser langsam ab. Die Ausbeute beträgt 92 g.

## Vorschrift B

### tert.Butyl-cyclohexencarboxaldehyd ($\Delta^3$tert.Butyl-tetrahydrobenzaldehyd)

tert.Butylbutadien und Acrolein wurden in molaren Mengen 6 bis 8 Stunden am Rückfluß erhitzt. Man erhält ein Gemisch aus etwa 70% 4-tert.Butyl-cyclohexen-3-carboxaldehyd-1 und etwa 30% 3-tert.Butyl-cyclohexen-3-carboxaldehyd-1 in ca. 80% Ausbeute. Kp. 112°C/28 mbar.

## Vorschrift C

Ein nach Vorschrift B hergestelltes Gemisch aus $\Delta^3$-4-tert.Butyl- und $\Delta^3$-3-tert.Butyl-tetrahydrobenzaldehyd wird in bekannter Weise mit Propionaldehyd kondensiert. Man erhält in 80% Ausbeute 2-Methyl-(4-tert.butyl-cyclohexen-3-yl)-propen-2-al-1 und 2-Methyl-(3-tert. butylcyclohexen-3-yl)-propen-2-al-1 im Verhältnis ca. 70 : 30. Kp. 153°C/30 mbar.

## Beispiel 1

73 g eines nach Vorschrift C hergestellten Gemisches wurde in 500 g Methanol gelöst, mit 40 g cis-2,6-Dimethylmorpholin versetzt und in Gegenwart von 7,8 g $\gamma$-$Al_2O_3$ mit 0,5% Pd in einem Rührautoklaven bei 40°C mit 50 bar $H_2$ umgesetzt, bis die Wasserstoffaufnahme unter 1 bar/h absank. Anschließend wurde noch bei 100°C und 50 bar $H_2$ 3 Stunden nachhydriert. Man arbeitet den Austrag destillativ auf und erhält in ca. 75% Ausbeute die Mischung von N-(3[4'-tert.-Butyl-cyclohexen-3'yl]-2-methyl-propyl-1)-cis-2,6-dimethylmorpholin mit einer entsprechenden 3'-tert.Butylverbindung im Verhältnis 70 : 30. Kp 142—144°C/1 mbar.

## Beispiel 2

Das Amingemisch aus Beispiel 1 wird in einem festen inerten Lösungsmittel durch Eingasen von trockenem HCl-Gas in das Hydrochlorid überführt, dieses aus Methanol umkristallisiert (Fp. 190°C) und das Salz mit Alkali wieder in die Base zerlegt. Man erhält reines N-(3[4'-tert.Butyl-cyclohexen-3'yl]-2-methyl-propyl-1)-cis-2,6-dimethylmorpholin. Kp. 125°C/0,25 mbar. Das 3'-tert.Butylisomere ist spektroskopisch nicht mehr nachweisbar.

## Beispiel 3

Ein nach Vorschrift C hergestelltes Gemisch wird mit Hefe hydriert. Ein sauberer, nicht sterilisierter Fermenter mit 6-l-Gesamtvolumen wird folgendermaßen beschickt:

| | |
|---|---|
| Vollentsalztes Wasser | 1,8 l |
| Saccharose | 90 g |
| Preßhefe (Deutsche Hefewerke) | 200 g |
| Gemisch nach Vorschrift C | 8 g in 30 ml Ethanol |
| Silicon-Antischaummittel | 2 g |

Fermentationsbedingungen sind:

| | |
|---|---|
| Temperatur: | 30° C |
| Drehzahl des Rührers: | 500 U/min |
| Belüftungsrate: | 1 VVM |
| Fermentationszeit: | 52,5 h |

Die Fermentation wird nach 52,5 h abgebrochen. Die Zellmasse wird durch Zentrifugation von der Nährlösung getrennt; beide Phasen werden je 3mal mit Methylenchlorid extrahiert. Die vereinigten Extrakte werden über $Na_2SO_4$ getrocknet und im Vakuum eingedampft. Der Rückstand wird destilliert. Man erhält ein (—)-drehendes Gemisch aus 2-Methyl-[4-tert.Butyl-cyclohexen-3-yl]-propanol-1 und 2-Methyl-[3-tert. Butyl-cyclohexen-3-yl]-propanol-1 im Verhältnis 70 : 30. Dieses Gemisch wird in das Tosylat überführt und mit cis-2,6-Dimethylmorpholin umgesetzt. Man erhält dasselbe Gemisch wie gemäß Beispiel 1, jedoch in optisch aktiver Form $\alpha_D^{20} = -2,6°$ (pur).

## Beispiel 4

Man verfährt wie in Beispiel 1 beschrieben und erhält so folgende fungizid wirksamen Amine (Gemische)

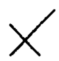 = tert. Butyl

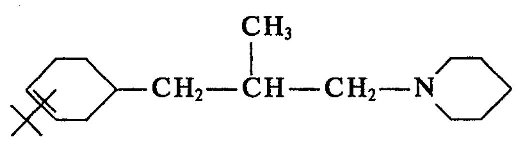

Kp. 191°C/17 mbar

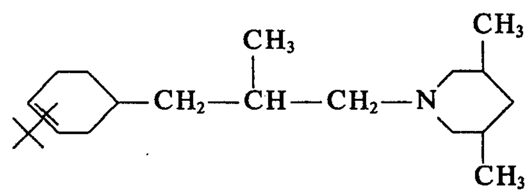

Kp. 203°C/12 mbar

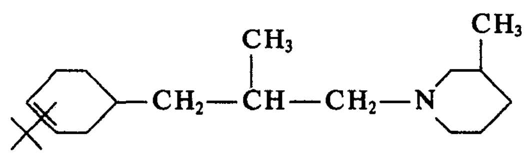

Kp. 144°C/0,4 mbar

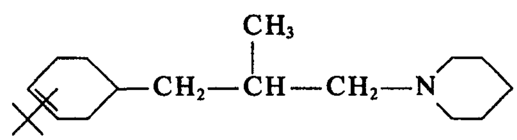

Kp. 118°C/0,1 mbar

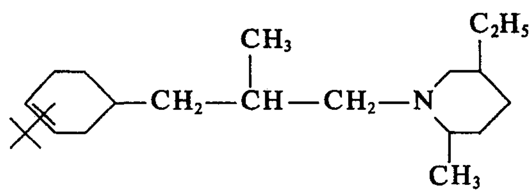

Kp. 212°C/14 mbar

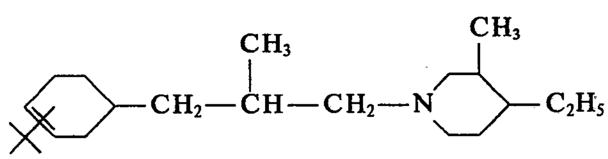

Kp. 171°C/3 mbar

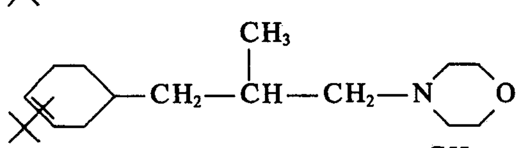

Kp. 184°C/8 mbar

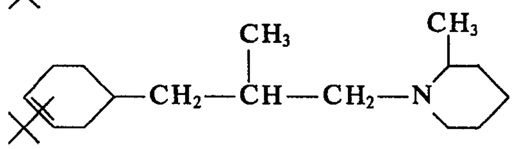

Kp. 160°C/5 mbar

Aus diesem Gemisch lassen sich die 4-tert. Butylverbindungen gemäß Beispiel 2 in reiner Form isolieren.

Die neuen Verbindungen sind insbesondere geeignet zur Bekämpfung von Pflanzenkrankheiten, z. B. Erysiphe graminis (echter Mehltau) an Getreide, Erysiphe cichoriacearum (echter Mehltau) an Kürbisgewächsen, Podosphaera leucotricha an Äpfeln, Uncinula necator an Reben, Erysiphe polygoni an Bohnen, Sphaerotheca pannosa an Rosen, Microsphaera querci an Eichen, Botrytis cinerea an Erdbeeren, Reben, Mycosphaerella musicola an Bananen, Puccinia-Arten (Rostpilze) an Getreide, Uromyces appendiculatus und U. phaseoli an Bohnen, Hemileia vastatrix an Kaffee und Rhizoctonia solani. Sie sind systematisch wirksam; sie werden sowohl über die Wurzeln als auch über die Blätter aufgenommen und im Pflanzengewebe transportiert.

Für den folgenden Versuch wurde zum Vergleich als bekannter Wirkstoff 4-[3-(p-tert.Butylcyclohexyl)-2-methyl-propyl]-cis-2,6-dimethyl-morpholin(A) (DE-OS 2 921 131) verwendet.

## Wirksamkeit gegen Rhizoctonia solani

Baumwollsamen der Sorte »Delta Pine« werden mit einer Beizmittelaufbereitung, die 40% (Gewichtsprozent) des zu prüfenden Wirkstoffes und 60% Talkum in der Verreibung enthält, in Mengen von 0,3 g je 100 g Körner gründlich eingestäubt. Die auf diese Weise behandelten Baumwollsamen werden in Töpfe eingelegt und mit Erde bedeckt, die zuvor künstlich mit dem Pilz Rhizoctonia solani beimpft worden ist. Nach 21-tägiger Versuchsdauer wird der Krankheitsbefall im Vergleich mit einem bekannten Fungizid und der nichtgebeizten Kontrolle beurteilt. Das Ergebnis des Versuches ergibt, daß 21 Tage nach dem Auflauf der Baumwollpflanzen die Mischungen entsprechend den Beispielen 1 und 3 eine gute fungizide Wirkung und die bekannte Verbindung (A) nur eine geringe fungizide Wirkung zeigt.

## Wirksamkeit gegen Weizenmehltau

Blätter von in Töpfen gewachsenen Weizenkeimlingen der Sorte »Jubilar« werden mit wäßrigen Emulsionen aus 80% (Gewichtsprozent) Wirkstoff und 20% Emulgiermittel (Na-Ligninsulfonat) besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

Als Ergebnis der Prüfung ergibt sich, daß die gemäß Beispiel 1 (Gemisch), 2 und 3 (Gemisch) hergestellten Wirkstoffe eine gute fungizide Wirkung haben.

Bei der Anwendung der Wirkstoffe zur Behandlung von Pflanzen gegen Pilzinfektionen liegen die Aufwandmengen zwischen 0,025 und 5 kg Wirkstoff/ha Fläche. Zum Oberflächenschutz von Bäumen oder Früchten können die Wirkstoffe auch in Verbindung mit Kunststoffdispersionen 0,25%ig bis 5%ig, bezogen auf das Gewicht der Dispersion, verwendet werden. Die Fungizide enthalten im allgemeinen zwischen 0,1 und 95 Gewichtsprozent Wirkstoff, vorzugsweise zwischen 0,5 und 90%.

Die Wirkstoffe eignen sich auch zur Bekämpfung holzzerstörender Pilze, wie Coniophora puteana, Poria vaporaria, Lenzites trabea und Polystictus versicolor. Lösemittelhaltige Holzschutzmittel-Formulierungen können beispielsweise 0,5 bis 2 Gewichtsprozent Wirkstoff enthalten.

Die Wirkstoffe können mit anderen bekannten Fungiziden gemischt werden. In vielen Fällen erhält man dabei eine Vergrößerung des fungiziden Wirkungsspektrums; bei einer Anzahl von Fungizidmischungen in den Gewichtsverhältnissen 1 : 10 bis 10 : 1 treten auch synergistische Effekte auf, d. h. die fungizide Wirksamkeit des Kombinationsproduktes ist größer als die der addierten Wirksamkeiten der Einzelkomponenten. Fungizide, die mit den erfindungsgemäßen Verbindungen kombiniert werden können, sind beispielsweise:

Dithiocarbamate und deren Derivate, wie
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfid,
Ammoniak-Komplex von Zink-(N,N-ethylen-bis-dithiocarbamat)
und
N,N'-Polyethylen-bis-(thiocarbomoyl)-disulfid,
Zink-(N,N'-propylen-bis-dithiocarbamat),
Ammoniak-Komplex von Zink-(N,N'-propylen-bis-dithiocarbamat)
und
N, N'-Polypropylen-bis-thiocarbamoyl)-disulfid;

heterocyclische Verbindungen, wie
N-Trichlormethylthio-tetrahydrophthalimid,
N-Trichlormethylthio-phthalimid,
N-(1,1,2,2-Tetrachlorethylthio)-tetrahydrophthalimid,
1-(Butylcarbamoyl)-2-benzimidazol-carbaminsäuremethylester,
2-Methyloxycarbonylamino-benzimidazol,
2,3-Dihydro-5-carboxanilido-6-methyl-1,4-oxathiin-4,4-dioxid,
2,3-Dihydro-5-carboxanilido-6-methyl-1,4-oxathiin,
5-Butyl-2-dimethylamino-4-hydroxy-6-methyl-pyrimidin,
1,2-Bis-(3-ethoxycarbonyl-2-thioureido)-benzol,
1,2-Bis-(3-methoxycarbonyl-2-thioureido)-benzol
und verschiedene andere Fungizide, wie
Dodecylguanidinacetat,
N-Dichlorfluormethylthio-N',N'-dimethyl-N-phenylschwefel-säurediamid,
2,5-Dimethyl-furan-3-carbonsäureanilid,
2,5-Dimethyl-furan-3-carbonsäure-cyclohexylamid,
2-Jodbenzoesäureanilid,
2-Brombenzoesäureanilid,
3-Nitro-isophthalsäure-diisopropylester,
1-(1,2,4-Triazolyl-1')-[1-(4'-chlorphenoxy)]-3,3dimethylbutan-2-on,
1-(1-Imidazolyl)-2-allyloxy-2-(2,4-dichlorphenyl)ethan,
Piperazin-1,4-diyl-bis-1-(2,2,2-Trichlorethyl)-formamid,
2,4,5,6-Tetrachlor-isophthalonitril,
1,2-Dimethyl-3,5-diphenyl-pyrazoliniummethylsulfat.

Die Anwendung erfolgt z. B. in Form von direkt versprühbaren Lösungen, Pulvern, Suspensionen oder Dispersionen, Emulsionen, Öldispersionen, Pasten, Stäubemittel, Streumitteln, Granulaten durch Versprühen, Vernebeln, Verstäuben, Verstreuen, Beizen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw. sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht. Die Salze können in Form ihrer wäßrigen Lösungen verwendet werden.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether. Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden. Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Zu den Mischungen oder Einzelwirkstoffen können Öle verschiedenen Typs, Herbizide, Fungizide, Nematozide, Insektizide, Bakterizide, Spurenelemente, Düngemittel, Antischaummittel (z. B. Silikone), Wachstumsregulatoren, Antidotmittel oder andere wirksame Verbindungen, zugesetzt werden.

Beispiel I

20 Teile des Wirkstoffs gemäß Beispiel 1 werden mit 12 Teilen Calciumsalz der Dodecylbenzolsulfonsäure, 8 Teile Fettalkoholpolyglykolether, 2 Teilen Natriumsalz eines Phenolsulfonsäure-harnstoffkondensats, 68 Teilen eines paraffinischen Mineralöls innig vermischt. Man erhält eine stabile ölige Dispersion.

Beispiel II

3 Gewichtsteile des Wirkstoffs gemäß Beispiel 2 werden mit 97 Gewichtsteilen feinteiligem Kaolin innig vermischt. Man erhält auf diese Weise ein Stäubemittel, das 3 Gewichtsprozent des Wirkstoffs enthält.

Beispiel III

30 Gewichtsprozent des Wirkstoffs gemäß Beispiel 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung mit guter Haftfähigkeit.

Beispiel IV

40 Gewichtsteile des Wirkstoffs gemäß Beispiel 1 werden mit 10 Teilen Natriumsalz eines Phenolsulfonsäure-formaldehyd-kondensats, 2 Teilen Kieselsäuregel und 48 Teilen Wasser innig vermischt. Man erhält eine stabile wäßrige Dispersion.

Beispiel V

20 Gewichsteile des Wirkstoffs gemäß Beispiel 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht.

Beispiel VI

20 Gewichtsteile des Wirkstoffs gemäß Beispiel 3 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht.

Beispiel VII

20 Gewichtsteile des Wirkstoffs gemäß Beispiel 1 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen.

**Patentansprüche pour les Etats contractants BE CH DE FR GB IT LI NL SE**

1. Cyclohexenderivat der Formel I

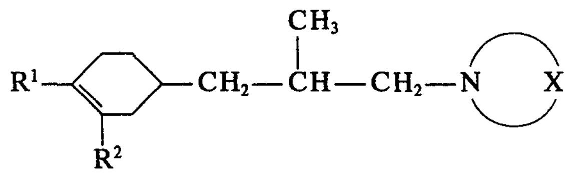

(I)

in der $R^1$ und $R^2$ voneinander verschieden sind und Wasserstoff oder eine tert.Butylgruppe bedeuten und N und X Glieder eines sechsgliedrigen heterocyclischen Ringes bedeuten, wobei X eine Alkylenkette mit fünf Gliedern ist, die gegebenenfalls ein Sauerstoffatom als Kettenglied enthält, und deren Alkylenreste gegebenenfalls ein- oder mehrfach durch Alkylreste mit 1 bis 4 Kohlenstoffatomen oder durch den Rest $-CH_2OH$ oder sein Acetyl- oder Propionylderivat substituiert sind, und ihre Salze.

2. Fungizid, enthaltend ein Cyclohexenderivat der Formel I gemäß Anspruch 1.

3. Fungizid, enthaltend einen festen oder flüssigen Trägerstoff und ein Cyclohexenderivat der Formel I gemäß Anspruch 1.

4. Verfahren zur Herstellung eines Fungizids, dadurch gekennzeichnet, daß man einen festen oder flüssigen Trägerstoff vermischt mit einem Cyclohexenderivat der Formel I gemäß Anspruch 1.

5. Verfahren zur Bekämpfung von Pilzen, dadurch gekennzeichnet, daß man die Pilze oder die vor Pilzbefall zu schützenden Gegenstände behandelt mit einem Cyclohexenderivat der Formel I gemäß Anspruch 1.

6. Verfahren zur Herstellung eines Cyclohexenderivats der Formel I gemäß Anspruch 1, dadurch gekennzeichnet, daß man eine Verbindung der Formel II

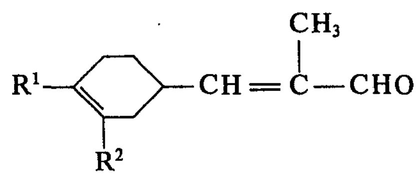

(II)

in der $R^1$ und $R^2$ die im Anspruch 1 genannten Bedeutungen haben, in Gegenwart eines Amins der Formel III

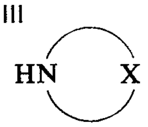

(III)

in der N und X die im Anspruch 1 genannten Bedeutungen haben, in Gegenwart eines Hydrierkatalysators mit Wasserstoff umsetzt.

7. N-(3-[4-tert.-Butyl-cyclohexen-3-yl]-2-methyl-propyl-1)-2,6-dimethylmorpholin.

8. N-(3-[4-tert.-Butyl-cyclohexen-3-yl]-2-methyl-propyl-cis-2,6-dimethylmorpholin.

9. Fungizid enthaltend N-(3-[4-tert.-Butyl-cyclohexen-3-yl]-2-methyl-propyl-1) -2,6-dimethylmorpholin.

10. Fungizid enthaltend N-(3-[4-tert.-Butyl-cyclohexen-3-yl]-2-methyl-propyl-cis-2,6-dimethylmorpholin.

**Patentansprüche für den Vertragsstaat AT**

1. Fungizid, enthaltend ein Cyclohexenderivat der Formel I

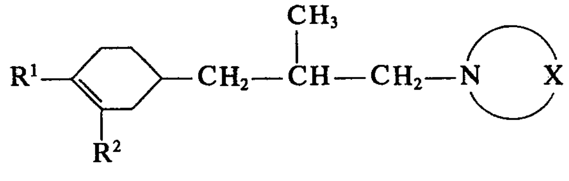

(I)

in der $R^1$ und $R^2$ voneinander verschieden sind und Wasserstoff oder eine tert.-Butylgruppe bedeuten und N und X Glieder eines sechsgliedrigen heterocyclischen Ringes bedeuten, wobei X eine Alkylenkette mit fünf Gliedern ist, die gegebenenfalls ein Sauerstoffatom als Kettenglied enthält, und deren Alkylenreste gegebenenfalls ein- oder mehrfach durch Alkylreste mit 1 bis 4 Kohlenstoffatomen oder

durch den Rest $-CH_2OH$ oder sein Acetyl- oder Propionylderivat substituiert sind, und ihre Salze.

2. Fungizid, enthaltend einen festen oder flüssigen Trägerstoff und ein Cyclohexenderivat der Formel I gemäß Anspruch 1.

3. Verfahren zur Herstellung eines Fungizids, dadurch gekennzeichnet, daß man einen festen oder flüssigen Trägerstoff vermischt mit einem Cyclohexenderivat der Formel I gemäß Anspruch 1.

4. Verfahren zur Bekämpfung von Pilzen, dadurch gekennzeichnet, daß man die Pilze oder die vor Pilzbefall zu schützenden Gegenstände behandelt mit einem Cyclohexenderivat der Formel I gemäß Anspruch 1.

5. Verfahren zur Herstellung eines Cyclohexenderivats der Formel I gemäß Anspruch 1, dadurch gekennzeichnet, daß man eine Verbindung der Formel II

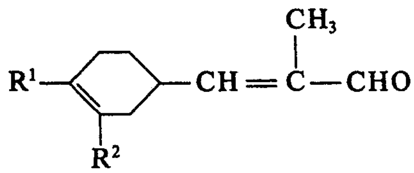 (II)

in der $R^1$ und $R^2$ die im Anspruch 1 genannten Bedeutungen haben, in Gegenwart eines Amins der Formel III

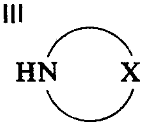 (III)

in der N und X die im Anspruch 1 genannten Bedeutungen haben, in Gegenwart eines Hydrierkatalysators mit Wasserstoff umsetzt.

6. Fungizid, enthaltend N-(3-4-tert.-Butyl-cyclohexen-3-yl-2-methyl-propyl-1)-2,6-dimethylmorpholin.

7. Fungizid, enthaltend N-(3-4-tert.-Butyl-cyclohexen-3-yl-2-methyl-propyl-cis-2,6-dimethylmorpholin.

**Claims for the Contracting States BE CH DE FR GB IT LI LU NL SE**

1. A cyclohexene derivative of the formula I

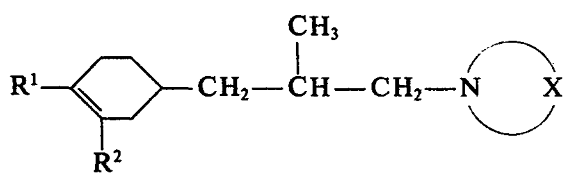 (I)

where $R^1$ and $R^2$ are different and each is hydrogen or tert.-butyl and N and X are members of a 6-membered heterocyclic ring in which X is an alkylene chain of five members, one of which may be oxygen, and where the alkylene radicals are unsubstituted or mono- or polysubstituted by alkyl of 1 to 4 carbon atoms or by $-CH_2OH$ or its acetyl or propionyl derivative, or a salt thereof.

2. A fungicide containing a cyclohexene derivative of the formula I as claimed in claim 1.

3. A fungicide containing a solid or liquid carrier and a cyclohexene derivative of the formula I as claimed in claim 1.

4. A process for the production of a fungicide, wherein a solid or liquid carrier is mixed with a cyclohexene derivative of the formula I as claimed in claim 1.

5. A process for combating fungi, wherein the fungi or the objects to be protected against fungus attack are treated with a cyclohexene derivative of the formula I as claimed in claim 1.

6. A process for the preparation of a cyclohexene derivative of the formula I as claimed in claim 1, wherein a compound of the formula II

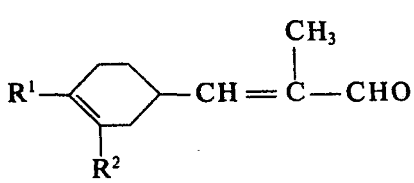 (II)

where $R^1$ and $R^2$ have the meanings given in claim 1, is reacted with hydrogen in the presence of an

amine of the formula III

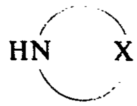

(III)

where N and X have the meanings given in claim 1, and in the presence of a hydrogenation catalyst.

7. N-(3-[4-tert-butyl-cyclohex-3-enyl]-2-methyl-prop-1-yl)-2,6-dimethylmorpholine.

8. N-(3-[4-tert-butyl-cyclohex-3-enyl]-2-methyl-propyl)-cis-2,6-dimethylmorpholine.

9. A fungicide containing N-(3-[4-tert-butyl-cyclohex-3-enyl]-2-methyl-prop-1-yl)-2,6-dimethylmorpholine.

10. A fungicide containing N-(3-[4-tert-butyl-cyclohex-3-enyl]-2-methyl-propyl)-cis-2,6-dimethylmorpholine.

**Claims for the Contracting State AT**

1. A fungicide containing a cyclohexene derivative of the formula I

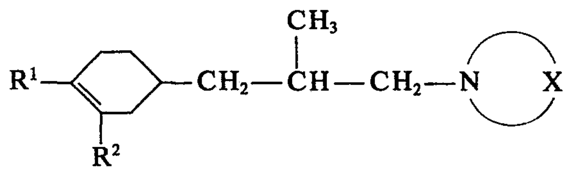

(I)

where $R^1$ and $R^2$ are different and each is hydrogen or tert.-butyl and N and X are members of a 6-membered heterocyclic ring in which X is an alkylene chain of five members, one of which may be oxygen, and where the alkylene radicals are unsubstituted or mono- or polysubstituted by alkyl of 1 to 4 carbon atoms or by $-CH_2OH$ or its acetyl or propionyl derivative, or a salt thereof.

2. A fungicide containing a solid or liquid carrier and a cyclohexene derivative of the formula I as defined in claim 1.

3. A process for the production of a fungicide, wherein a solid or liquid carrier is mixed with a cyclohexene derivative of the formula I as defined in claim 1.

4. A process for combating fungi, wherein the fungi or the objects to be protected against fungus attack are treated with a cyclohexene derivative of the formula I as defined in claim 1.

5. A process for the preparation of a cyclohexene derivative of the formula I as defined in claim 1, wherein a compound of the formula II

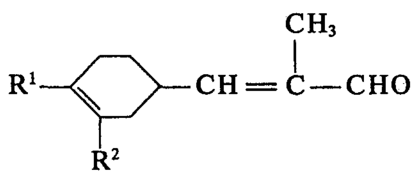

(II)

where $R^1$ and $R^2$ have the meanings given in claim 1, is reacted with hydrogen in the presence of an amine of the formula III

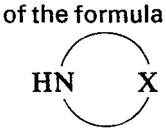

(III)

where N and X have the meanings given in claim 1, and in the presence of a hydrogenation catalyst.

6. A fungicide containing N-(3-[4-tert-butyl-cyclohex-3-enyl]-2-methyl-prop-1-yl)-2,6-dimethylmorpholine.

7. A fungicide containing N-(3-[4-tert-butyl-cyclohex-3-enyl]-2-methyl-propyl)-cis-2,6-dimethylmorpholine.

**Revendications pour les Etats contractants BE CH DE FR GB IT LI LU NL SE**

1. Dérivé du cyclohexène de la formule I

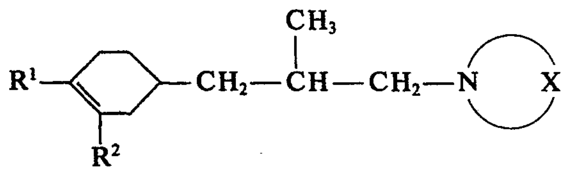

(I)

dans laquelle $R^1$ et $R^2$, différents l'un de l'autre, désignent chacun un atome d'hydrogène ou un groupe butyle tertiaire et N et X sont des chaînons d'un noyau hétérocyclique hexagonal, X représentant une chaîne alkylène avec cinq chaînons, comprenant éventuellement un atome d'oxygène comme chaînon et dont les groupes alkylène peuvent être mono- ou pluri-substitués par des radicaux alkyle en $C_1$ à $C_4$ ou par le groupe $-CH_2OH$ ou le dérivé acétyle ou propionyle de celui-ci, ainsi que ses sels.

2. Fongicide, contenant un dérivé du cyclohexène de la formule I selon la revendication 1.

3. Fongicide, contenant un dérivé du cyclohexène de la formule I selon la revendication 1, ainsi qu'un support solide ou liquide.

4. Procédé de préparation d'un fongicide, caractérisé en ce que l'on mélange un dérivé du cyclohexène de la formule I selon la revendication 1 avec un support solide ou liquide.

5. Procédé de lutte contre les champignons, caractérisé en ce que l'on traite les champignons ou les objets à protéger des champignons avec un dérivé du cyclohexène de la formule I selon la revendication 1.

6. Procédé de préparation d'un dérivé du cyclohexène de la formule I selon la revendication 1, caractérisé en ce que l'on fait réagir un composé de la formule II

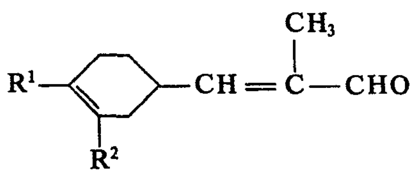

(II)

dans laquelle $R^1$ et $R^2$ possèdent les significations définiens dans la revendication 1, en présence d'une amine de la formule III

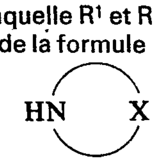

(III)

dans laquelle N et X possèdent les significations définies dans la revendication 1, et en présence d'un catalyseur d'hydrogénation avec l'hydrogène.

7. La N-(3-[4-tert.-butyl-cyclohexén-3-yl]-2-méthyl-propyl-1)-2,6-diméthyl-morpholine.

8. La N-(3-[4-tert.-butyl-cyclohexén-3-yl]-2-méthyl-propyl-cis) -2,6-diméthyl-morpholine.

9. Fongicide, contenant de la N-(3-[4-tert.-butyl-cyclohexén-3-yl]-2-méthyl-propyl-1)-2,6-diméthyl-morpholine.

10. Fongicide, contenant de la N-(3-[4-tert.-butyl-cyclohexén-3-yl]-2-méthyl-propyl-cis)-2, 6-diméthyl-morpholine.

**Revendications pour l'Etat contractant AT**

1. Fongicide, contenant un dérivé du cyclohexène de la formule I

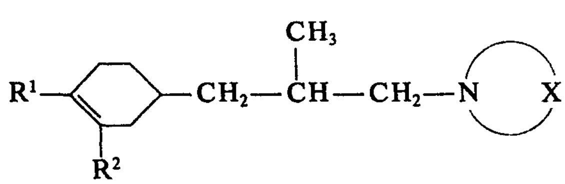

(I)

dans laquelle $R^1$ et $R^2$, différents l'un de l'autre, désignent chacun un atome d'hydrogène ou un groupe butyle tertiaire et N et X sont des chaînons d'un noyau hétérocyclique hexagonal, X représentant une chaîne alkylène avec cinq chaînons, comprenant éventuellement un atome d'oxygène comme chaînon et dont les groupes alkylène peuvent être mono- ou pluri-substitués par des radicaux alkyle en $C_1$ à $C_4$

ou par le groupe $-CH_2OH$ ou le dérivé acétyle ou propionyle de celui-ci, ou ses sels.

2. Fongicide, contenant un dérivé du cyclohexène de la formule I selon la revendication 1, ainsi qu'un support solide ou liquide.

3. Procédé de préparation d'un fongicide, caractérisé en ce que l'on mélange un dérivé du cyclohexène de la formule I selon la revendication 1 avec un support solide ou liquide.

4. Procédé de lutte contre les champignons, caractérisé en ce que l'on traite les champignons ou les objets à protéger des champignons avec un dérivé du cyclohexène de la formule I selon la revendication 1.

5. Procédé de préparation d'un dérivé du cyclohexène de la formule I selon la revendication 1, caractérisé en ce que l'on fait réagir un composé de la formule II

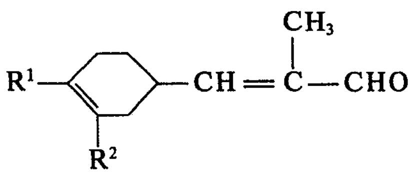

(II)

dans laquelle $R^1$ et $R^2$ possèdent les significations définies dans la revendication 1, en présence d'une amine de la formule III

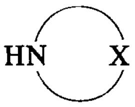

(III)

dans laquelle N et X possèdent les significations définies dans la revendication 1, et en présence d'un catalyseur d'hydrogénation avec l'hydrogène.

6. Fongicide, contenant de la N-(3-[4-tert.-butyl-cyclohexén-3-yl]-2-méthyl-propyl-1)-2,6-diméthylmorpholine.

7. Fongicide, contenant de la N-(3-[4-tert.-butyl-cyclohexén-3-yl]-2-méthyl-propyl-cis)-2,6-diméthylmorpholine.